Europäisches Patentamt

European Patent Office

Office européen des brevets

(11) Veröffentlichungsnummer: 0 025 005
A1

(12) **EUROPÄISCHE PATENTANMELDUNG**

(21) Anmeldenummer: 80730057.9

(22) Anmeldetag: 18.08.80

(51) Int. Cl.³: **H 01 L 41/08**
F 04 B 43/04, A 61 M 5/14

(30) Priorität: 18.08.79 DE 2933799

(43) Veröffentlichungstag der Anmeldung:
11.03.81 Patentblatt 81/10

(84) Benannte Vertragsstaaten:
AT CH DE FR GB IT LI NL SE

(71) Anmelder: Schaldach, Max, Prof. Dr. Ing.
Turnstrasse 5
D-8520 Erlangen(DE)

(72) Erfinder: Schaldach, Max, Prof. Dr.-Ing.
Turnstrasse 5
D-8520 Erlangen(DE)

(72) Erfinder: Schubert, Walter
Gebbertstrasse 52
D-8520 Erlangen(DE)

(72) Erfinder: Thull, Roger, Dr.
Köhlerstrasse 8
D-8551 Hemmhofen(DE)

(74) Vertreter: Christiansen, Henning, Dipl.-Ing.
Unter den Eichen 108a
D-1000 Berlin 45(DE)

(54) Vorrichtung zum Fördern und Dosieren kleinster Flüssigkeitsmengen.

(57) Bei dieser Vorrichtung zum Fördern und Dosieren kleinster Flüssigkeitsmengen, insbesondere zur Anwendung für den menschlichen oder tierischen Körper, bestehend aus einer piezokeramisch beschichteten Membran, die mit ihrem Rand allseitig an eine starre Wand angrenzt, wobei die so gebildete Pumpenkammer eine durch entsprechende Mittel verschließbare Durchlaßöffnung aufweist, ist die Durchlaßöffnung bei spannungsloser piezokeramischer Beschichtung (3, 4, 3', 4') durch die dabei an der starren Wand flach anleigende Membran (7, 7') unabhängig von den Verschlußmitteln der Durchlaßöffnung dichtend abgeschlossen, die Membran eine Öffnung (11, 11') mit Sperrmitteln (9) aufweist, deren Durchlässigkeit entgegengesetzt zur Förderrichtung gering ist, bezogen auf die Durchlässigkeit der unverschlossenen Durchlaßöffnung (13, 13') und die Sperrmittel (9) entfernt von dem Ort der Durchlaßöffnung (13) bei flach anliegender Membran (7, 8) angeordnet.

./...

# FIG.7

Seite 1

Prof. Dr.-Ing. Max Schaldach          18. August 1980
Turnstr. 5

8520 Erlangen

SCH79.2-EU

---

## Vorrichtung zum Fördern und Dosieren kleinster Flüssigkeitsmengen

---

B e s c h r e i b u n g

Die Erfindung bezieht sich auf eine Vorrichtung zum Fördern und Dosieren kleinster Flüssigkeitsmengen, insbesondere zur Anwendung für den menschlichen oder tierischen Körper, bestehend aus einer piezokeramisch beschichteten Membran, die mit ihrem Rand allseitig an eine starre Wand angrenzt, wobei die so gebildete Pumpenkammer eine durch entsprechende Mittel verschließbare Durchlaßöffnung aufweist.

Bei der Therapie verschiedener Krankheiten, wie z.B. des Diabetes mellitus (Zuckerkrankheit), ist es notwendig, kleinste Medikamentendosen über große Zeitintervalle hinweg dem menschlichen Körper zuzuführen. Dabei kommt bei einigen Krankheiten, wie gerade bei der Zuckerkrankheit, erschwerend hinzu, daß der Medikamentenbedarf zeitlichen Schwankungen unterliegt, so daß eine zeitlich unabhängige Dosierung mit konstanter Förderrate keine ausreichende Lösung darstellt. So ist der Insulinbedarf des Diabetikers, bedingt durch den Rhythmus der Mahlzeiten, durch Arbeit, Ruhe usw., während des Tagesablaufs großen Schwankungen unterworfen. Die Höhe der notwendigen Raten liegt bei einer Insulinkonzentration von 500 IE/ml in der Größenordnung von einigen Mikrolitern pro Stunde.

Es sind bereits mehrere Vorrichtungen zur Infusion von Flüssigkeiten in den menschlichen Körper bekannt. Beispielsweise ist in Trans. Am. Soc. Artif. Intern. Organs, XXIII, 13 bis 16, 1977 eine Anordnung beschrieben, bei der zur Förderung ein Überdruckspeicher und zur Dosierung eine Kapillare vorgesehen sind. Dieses Gerät liefert jedoch nur in der Lage, eine konstante Förderrate zu erzeugen, so daß zeitliche Schwankungen im Medikamentenbedarf der Patienten unberücksichtigt bleiben müssen.

Eine Verbesserung dieses Konzepts wird beschrieben in Trans. Am. Soc. Artif. Intern. Organs XXIV, 229 bis 231, 1978. Auch hierbei wird dasselbe Förderprinzip verwendet, zur Dosierung dient jedoch eine Parallelschaltung, bestehend aus Kapillare und Magnetventil, mit einem nachgeschalteten Strömungswiderstand. Das Ventil wird über einen Magneten transcutan, also von außerhalb des Körpers durch die Haut, betätigt. Die Betätigung durch den Patienten selbst ist diesem auf die Dauer nicht zumutbar, so daß zusätzlich auf ein zweites, extern zu tragendes Gerät zurückgegriffen werden muß. Eine Steuerung des Ventils durch das implantierte System aufgrund von durch im Körper angeordnete Meßfühler ermittelte Werte (z.B. implantierbarer Glukosesensor) ist hierbei in jedem Fall ausgeschlossen. Des weiteren besteht bei der Anwendung derartiger Ventile die Gefahr, daß ein solches bei einem eventuellen Versagen offen bleibt, der Medikamentenfluß nicht mehr unterbrochen werden kann und somit die Sicherheit des Patienten beeinträchtigt ist.

Ein anderes Förder- und Dosierprinzip ist in Trans. Am. Soc. Artif. Intern. Organs XXI, 516 bis 520, 1975 beschrieben. Hierbei wird eine Membranpumpe verwendet, bestehend aus einem Haltering und zwei auf der Außenseite der Pumpe angeordneten mit Piezokeramik beschichteten Metallmembranen. Ein elektromagnetisches 3-Wege-Ventil dient zur Steuerung des Medikamentenflusses. Aufgrund des hohen Stromverbrauchs solcher Ventile und der bereits erwähnten Gefahr des Versagens scheidet eine Verwendung dieses Konzepts für ein implantierbares System aus.

Ein ähnliches Konzept, wie es der obengenannten Gattung entspricht, wird in in IEEE Transactions on Sonics and Ultrasonics SU-25, 3, 153 bis 156, 1978 vorgestellt. Hierbei werden anstelle des elektromagnetisch betätigten Ventils rechteckige, einseitig eingespannte, bimorphe Piezokeramikplättchen verwendet, die mit ihrem freien Ende im nicht angeregten Zustand die Verbindungsbohrungen Vorratsbehälter-Pumpe bzw. Pumpe-Ausflußkatheter verschließen. Die Pumpe selbst entspricht der bei der zuvor dargestellten Anordnung verwendeten, besitzt jedoch nur eine Metallmembran, die auf der nicht mit dem Medikament in Berührung kommenden Seite mit bimorphen Piezokeramikscheiben beklebt ist.

Alle aufgeführten Membranpumpen mit Piezokeramikantrieb zeigen eine starke Abhängigkeit der Förderrate vom herrschenden Gegendruck. Dies läßt die Verwendung für implantierbare medizinische Geräte, beispielsweise als Organersatz wie im Falle des künstlichen Pankreas, fragwürdig erscheinen, da z.B. durch Verstopfung des Katheters eine Druckerhöhung beim Ausstoßen auftreten kann, die somit zu einer Verringerung des ausgestoßenen Flüssigkeitsvolumens führt.

Der in Anspruch 1 angegebenen Erfindung liegt die Aufgabe zugrunde, ein Gerät der obengenannten Art zu entwickeln, das in der Lage ist, kleinste und dabei exakt dosierte Flüssigkeitsvolumina bei kleinen Ansprechzeiten unabhängig von im umgebenden Körpermilieu auftretendem Gegendruck zu fördern, wobei eine hohe Verschleißsicherheit gegeben und die Patientensicherheit zu jeder Zeit gewährleistet sein muß.

SCH79.2-EU          Seite 5

Die Erfindung beruht auf der Erkenntnis, daß die Membran im Ruhezustand eine zusätzliche die Betriebssicherheit erhöhende Dichtung bilden kann, wodurch sich unter vorteilhafter Einbeziehung weiterer Bauelemente in die Membran die Möglichkeit einer besonders kompakten Bauform ergibt.

Bei einer ersten bevorzugten Ausführungsform wird also mit Hilfe zweier beidseitig mit Piezokeramik beklebter Membranen zusammen mit einer feststehenden Trennwand bei jedem Pumpzyklus ein definiertes Volumen aus dem Vorratsbehälter entnommen und an den Körper abgegeben, ohne daß weitere Ventile erforderlich wären. Bei einer anderen vorteilhaften Weiterbildung wird eine derart beklebte Membran zusammen mit einer feststehenden Trennwand verwendet, wobei ein selbstschließendes Ventil die Funktion einer Rückflußdichtung übernimmt. Das Ventil benötigt dabei keine elektrische Energie und auch im Falle eines Versagens kann keine Flüssigkeit aus dem Vorratsbehälter unkontrolliert austreten.

Die Durchlaßöffnung in der Membran ist vorteilhafterweise als Strömungswiderstand ausgebildet, wofür sich bevorzugt ein Membranfilter eignet.

Durch die kompakte Bauform ist die Vorrichtung in beiden Ausführungen sowohl implantiert als auch bei externer Anwendung vielseitig verwendbar.

Besonders vorteilhaft sind neben der besonders kleinen Baugröße das geringe Gewicht, der niedrige Verbrauch elektrischer Energie und ein hohes Maß an Sicherheit für den Patienten, da das System in seiner Ruhestellung sicher geschlossen ist, wodurch keine Flüssigkeit aus dem Körper in das Vorratsgefäß oder umgekehrt gelangen kann. Das Fördervolumen wird allein von der Durchbiegung der Membran bestimmt, die proportional zu der an die Piezokeramik angelegten Spannung ist, und ist somit nur von dieser Spannung abhängig. Die Pumpenkammer wird damit also allein durch den zwischen Membran und starrer Wand gebildeten Raum bestimmt, bei dem die innerhalb dieses Raums vorhandene Menge praktisch restlos gefördert wird. Die Dosierung läßt sich damit äußerst präzise und unabhängig von äußeren Einflüssen bestimmen. Bei Verwendung von Membranen mit zweiseitiger piezokeramischer Beschichtung ist die Durchbiegung und damit das Fördervolumen pro Pumpenhub vergrößert.

Vorteilhafte Ausführungsbeispiele zu den angeführten Lösungen sind in den Fign. 1 bis 6 dargestellt und werden nachfolgend näher beschrieben. Es zeigen:

Figuren 1 bis 4 eine Schnittdarstellung eines bevorzugten Ausführungsbeispiels zu der ersten Lösung in verschiedenen Arbeitsphasen,

Figuren 5 und 6 eine entsprechende Darstellung eines Ausführungsbeispiels zu der zweiten Lösung in zwei Arbeitsphasen und

Figur 7 die Anwendung der in den Figuren 1 bis 4 wiedergegebenen Ausführungsform bei einem künstlichen Pankreas.

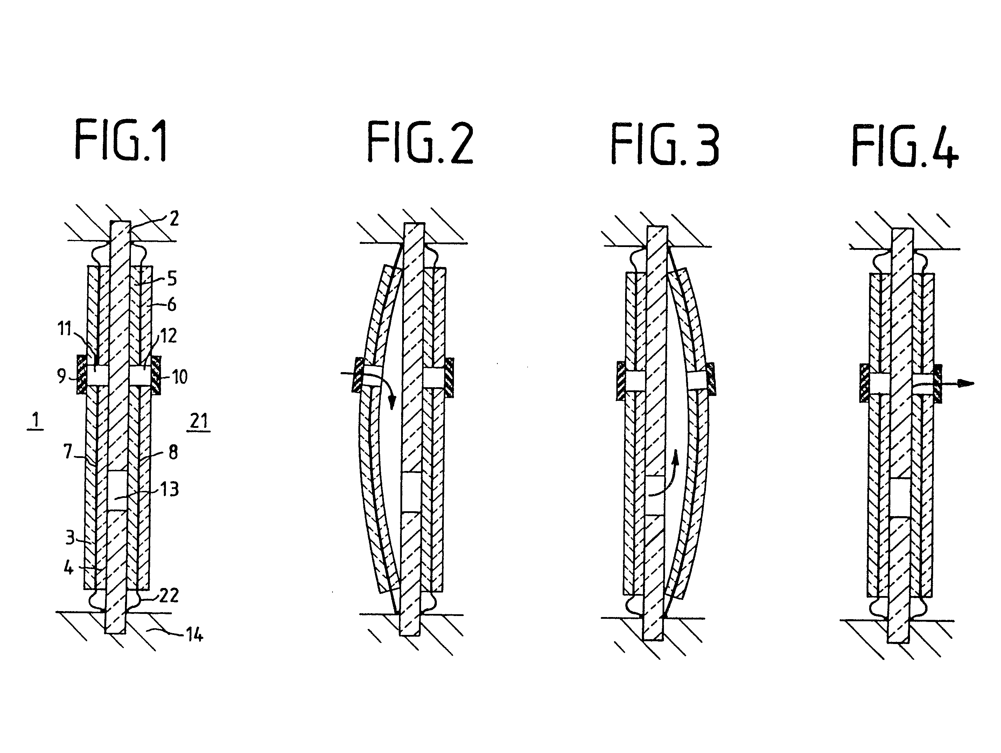

Bei dem in Fig. 1 wiedergegebenen Ausführungsbeispiel der erfindungsgemäßen Förder- und Dosiereinheit, ist in der Figur links ein - nicht vollständig dargestellter - Vorratsbehälter 1 für das zu dosierende Medikament angeordnet, während es auf der rechten Seite das Körpermilieu 21 angrenzt bzw. an einen Ausflußkatheter angeschlossen ist.

Auf beiden Seiten einer aus Keramik bestehenden Trennwand 2, die aber auch aus jedem anderen körper- und medikamentenverträglichen Werkstoff hergestellt sein kann, befindet sich je eine Membran 7 und 8, die bevorzugt aus Metall gefertigt ist. Die beiden Membranen können aber auch aus einem medikamenten- und körperverträglichen Kunststoff wie PTFE, etc. hergestellt sein. Sie tragen beidseitig je eine aufgeklebte Scheibe 3 bis 6, die aus piezokeramischen Werkstoffen besteht. Die Scheiben sind jeweils auf ihren planen Flächen mit einer leitenden Schicht versehen. Beim Anlegen einer Spannung zwischen diesen Flächen dehnen sie sich in radialer Richtung aus und die damit verbundene Membran erfährt eine entsprechende Wölbung.

Die Membranen weisen jeweils eine zwischen der aufgeklebten Keramik und der Einspannung 14 tangential umlaufende, eingeprägte Sicke 22 auf, welche dazu dient, eine optimale Durchbiegung der Membran durch aus der Sicke nachfließendes Material bei radialer Dehnung bzw. Kontraktion der piezokeramischen Scheiben zu gewährleisten. Die beidseitig aufgeklebten Scheiben bewirken eine größere Durchbiegung der Membran im Vergleich zu einer nur auf einer Seite mit Piezokeramik versehenen Membran. Des weiteren übernehmen die beiden inneren Keramikscheiben 4, 5 zusammen mit der Trennwand 2 eine Dichtungswirkung.

Auf die beiden, die Keramikscheiben 12 und die dazwischenliegende Membran durchdringenden Bohrungen 11 sind zwei an
der Außenseite aufgesetzte Membranfilter 9, 10 angebracht.
Diese bestehen bevorzugt aus PTFE mit einer Porosität von
2/um. Es können aber auch andere medikamenten- und körperverträgliche Werkstoffe verwendet werden.

Die Membranfilter dienen sowohl als Strömungswiderstand
als auch als bakteriologische Filter. Versetzt zu den beiden Bohrungen 11, 12 ist in der Trennwand 2 eine weitere
Bohrung 13 angeordnet.

Die beiden Membranen 7 und 8 mit den aufgeklebten Keramikscheiben 3 bis 6 sind beidseitig und die Bohrungen 11 und
12 innen mit einem Überzug aus Tetrafluoräthylen versehen.
Es eignen sich aber auch andere Schutzüberzüge, wenn sie
den medizinischen Anforderungen genügen. Ebenso kann die
Trennscheibe 2 entweder vor oder nach dem Zusammenbau und
die Einspannung 14 zusammen mit den Membranen entsprechend
überzogen werden.

Die zu dosierende Flüssigkeit befindet sich in dem Vorratsbehälter 1. Sie steht bevorzugt unter einem gewissen
Druck, der entweder dadurch aufgebracht wird, daß der Behälter als Überdruckspeicher ausgebildet ist, oder aber
der Druck wird mittels Federkraft oder ähnl. erzeugt.

Durch den Überdruck im Vorratsgefäß 1 wird die Membran 7,
auch ohne daß eine elektrische Spannung an die Piezokeramik 3, 4 angelegt ist, dichtend an die Bohrung 13 angepresst, so daß die Trennwand 2 abgeschlossen ist. Einen weiteren Abschluß zwischen Vorratsgefäß 1 und dem Körpermilieu 21 bildet die äußere Membran 8, wobei hier durch eine Vorspannung der Membran ein zusätzlicher Anpreßdruck zwischen Membran 8 und Trennscheibe 2 hervorgerufen werden kann. (Falls das Innere des Vorratsgefäßes 1 im Betrieb nicht unter Druck stehen soll, kann entsprechend auch die Membran 7 mit einer Vorspannung versehen werden.) Durch diese Konstruktion ist unter allen Umständen verhindert, daß durch Versagen der Steuerung der piezokeramischen Antriebe die im Vorratsgefäß aufbewahrte Flüssigkeit ungehindert Zutritt zum Körper erhält. Auf diese Weise gewährleistet das System eine optimale Patientensicherheit.

Werden die Piezokeramikscheiben 3, 4 durch einen Rechteckimpuls (Fig. 2 - die Bezugszeichen der Bauelemente in den Figuren 3 bis 4 ergeben sich aus Fig. 1) gleicher Größe aber umgekehrter Polarität angeregt, so biegen sie sich zusammen mit der dazwischenliegenden Membran 7 durch und es entsteht ein Hohlraum zwischen Membran 7 und Trennwand 2. Da die Öffnung 13 durch die Membran 8 weiterhin verschlossen ist, strömt die Medikamentenflüssigkeit aus dem Vorratsbehälter 1 durch das Membranfilter 9 und die Bohrung 11, gegebenenfalls noch unterstützt durch den oben erwähnten Überdruck, in den entstehenden Hohlraum und füllt diesen aus (Pfeil in Fig. 2).

Wird ein entsprechender Impuls unmittelbar anschließend an die Keramikscheiben 5, 6 der Membran 8 gelegt (Fig. 3), so biegt sich diese nach außen. Durch das Zusammenwirken von Durchbiegung der Membran 8 und gleichzeitigem Entspannen der Membran 7 wird das zwischen dieser und der Trennwand 2 befindliche Flüssigkeitsvolumen durch die Bohrung 13 in den entstandenen Raum zwischen Membran 8 und Trennwand 2 gedrückt (Pfeil in Fig. 3). Die Membranfilter 9, 10 verhindern durch ihren im Vergleich zu der Bohrung 13 großen Strömungswiderstand, daß in dieser Zeitspanne Flüssigkeit von außen, d.h. aus dem Vorratsgefäß oder dem Körpermilieu bzw. einer an das Filter 10 angesetzten Kanüle eindringt. Auf diese Weise wird ein definiertes Volumen aus dem Vorratsbehälter entnommen. Das Volumen wird dabei durch einen Speicher gebildet, der aus der zwischen Membran 7 und Trennwand 2 entstehenden Kammer besteht.

Beim Entspannen (Fig. 4), was durch ein entgegengesetztes Polarisieren der Keramikscheiben 5, 6 unterstützt werden kann, wird die Flüssigkeit anschließend in das Körpermilieu ausgestoßen, da die Bohrung 13 jetzt durch die Membran 7 verschlossen ist (Pfeil in Fig. 4). Die in der Bohrung 12 zurückbleibende Flüssigkeitsmenge wird durch das Membranfilter 10, das bevorzugt aus PTFE hergestellt ist, gegenüber dem Körpermilieu vor Kontamination geschützt. Die Spannungsimpulse werden durch - nicht dargestellte - Spannungserzeugemittel hervorgerufen, welche die notwendigen Spannungsimpulse entgegengesetzter Polarität in abwechselnder Folge abgeben können, wie es sich aus der vorangehenden Beschreibung ergibt. Durch Umkehrung der Reihenfolge der Ansteuerung der Membranen läßt sich die Pumprichtung für andere Anwendungsfälle ohne weiteres umkehren.

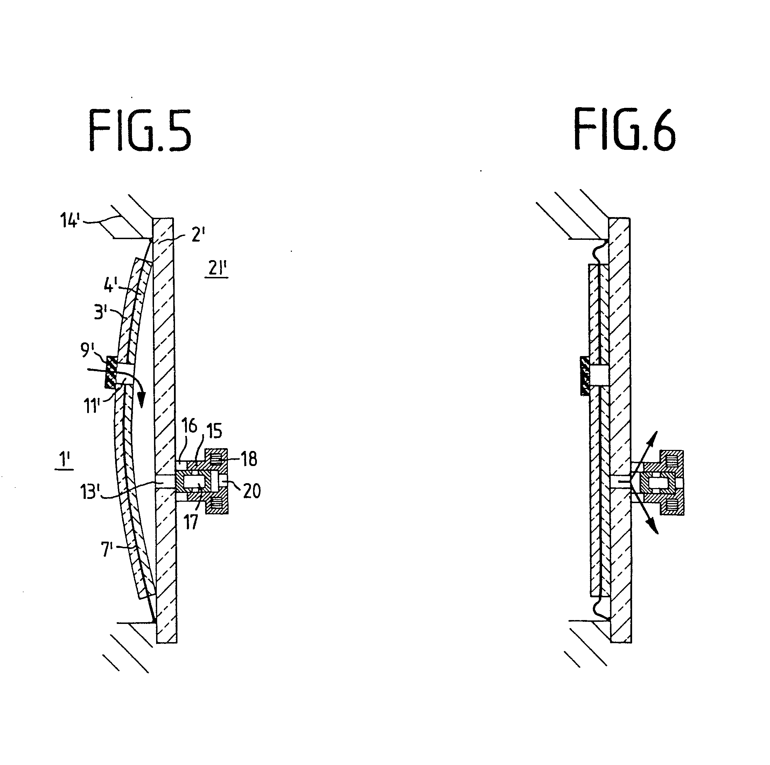

Eine andere Lösungsmöglichkeit zeigen die Fign. 5 und 6. Bauelemente, die den im Vorangehenden beschriebenen Figuren entsprechen, sind mit übereinstimmenden Bezugszeichen versehen, die zusätzlich ein " ' " enthalten. Im Gegensatz zu der in den Fign. 1 bis 4 dargestellten Vorrichtung ist die Membran 8 bei dieser Vorrichtung durch ein selbstschließendes Ventil ersetzt, das am Ende einer Bohrung 13' angebracht ist.

Die in Fig. 5 wiedergegeben Arbeitspostion entspricht derjenigen, die für die zuvor beschriebene Ausführung in Fig. 2 dargestellt ist. Durch Anlegen einer Gleichspannung entgegengesetzter Polarität an die Keramikscheiben 3', 4' biegt sich die Membran 7' von der Trennwand 2' weg. Das entstehende Volumen wird durch das Membranfilter 9' und die Bohrung 11' hindurch mit Flüssigkeit aus dem Vorratsbehälter 1' gefüllt (Pfeil in Fig. 5). Der Ausgang 13' zur Körperseite hin ist durch das Ventil verschlossen. Es ist in den Fign. 5 und 6 in schematischer Darstellung wiedergegeben und besteht aus dem Ventilgehäuse 15, mit Öffnungen 16 zum Flüssigkeitsaustritt, hergestellt aus einem körperverträglichen Werkstoff oder beschichtet mit einem solchen Werkstoff, und versehen mit einer Bohrung 20 zum Volumenausgleich bei der Bewegung des Ventilkörpers 17. In dem Ventilgehäuse 15 ist ein Ringdauermagnet 18 angeordnet, der beispielsweise aus einem Werkstoff auf der Basis Co/seltene Erden hergestellt ist. Der Ventilkörper 17 besteht aus einem aus einem körperverträglichen Werkstoff, wie z.B. PTFE-beschichteten Stabmagneten, der in bezug auf den Ringmagneten umgekehrt gepolt montiert ist, so daß das Ventil in Ruhestellung, d.h. ohne äußere Krafteinflüsse, durch die Abstoßungskräfte zwischen den beiden Magneten in geschlossenem Zustand gehalten ist.

Nach Abschalten der angelegten Spannung (Fig. 6) oder kurzzeitigem Anlegen einer Spannung umgekehrter Polarität schwingt die Membran in ihrer Ruhestellung zurück. Durch den Druck, den hierbei das zwischen der Membran 7' und der Trennwand 2' gespeicherte Flüssigkeitsvolumen auf den Ventilkörper 17 ausübt, wird dieser gegen die Magnetkraft aus seiner Ruhelage wegbewegt, und die Flüssigkeit in das Körpermilieu bzw. in eine an die Öffnungen 13' angeschlossene Kanüle gedrückt (Pfeil in Fig. 6). Selbst bei einem Versagen des selbstschließenden Ventils ist die Patientensicherheit unbedingt gewährleistet, da die Membran 7' die Ausflußöffnung 13' verschließt.

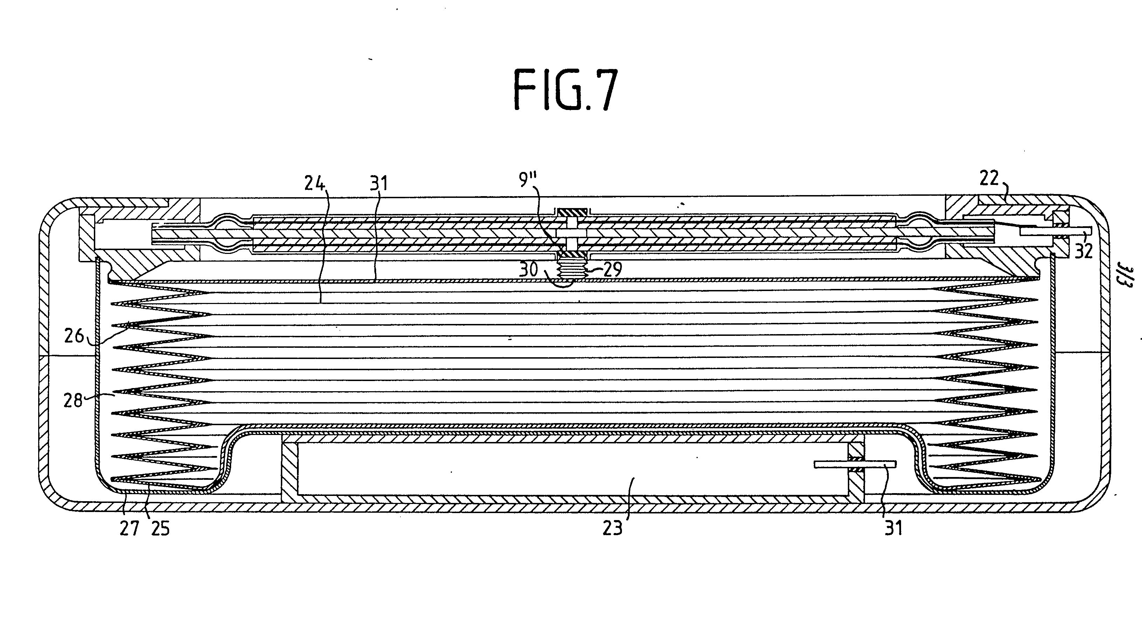

In Fig. 7 ist eine Ausführung der erfindungsgemäßen Dosiereinrichtung dargestellt, wie sie in einem implantierbaren künstlichen Pankreas Verwendung findet. Innerhalb eines hermetisch dicht gekapselten Metallgehäuses 22 ist neben einem separaten Gehäuse 23 für die elektronische Steuereinheit ein Insulinvorratsbehälter 24 angebracht, dessen Behälter 25 mit einer faltenbalgartigen Wandung 26 innerhalb eines weiteren Behälters 27 eingekapselt ist, der eine Treibgasfüllung 28 aufweist, welche die Tendenz hat, durch Druck auf den Vorratsbehälter 24 Insulin durch eine mit einem elastischen Verbindungsschlauch 29 abgeschlossene Öffnung 30 einer oberen Wandung 31 des Vorratsbehälters 24 gegen die mit einem Membranfilter 9" der Durchlaßöffnung der Membranpumpe zu drücken, die bezüglich Aufbau und Funktion im wesentlichen der in den Fign. 1 bis 4 dargestellten Anordnung entspricht. Der Verbindungsschlauch 29 ist elastisch ausgeführt, so daß er der Auslenkung der Membran nur einen geringen Widerstand entgegensetzt. Von besonderer Bedeutung bei der dargestellten Anordnung ist dabei, daß der im Insulinvorratsbehälter vorhandene Druck nicht auf die gesamte Fläche der Membranpumpe einwirkt, sondern nur im Bereich des Durchlasses 9" ausgeübt wird. Damit bleibt die für die Durchbiegung der inneren Membran bei jedem Pumpenhub jeweils erforderliche Kraft so klein, daß sie auch die in einer implantierbaren Einheit vorhandenen verhältnismäßig kleinen Energiereserven nicht überfordert.

Die erfindungsgemäße Pumpe bildet bei der dargestellten Anordnung einen Teil des hermetisch abgedichteten äußeren Gehäuses, so daß keine weiteren Probleme für die Gehäusedurchführung eines Auslasses für die abgegebene Insulinmenge bestehen. Die gewählte mehrfache Kapselung steht im übrigen in Übereinstimmung mit der Forderung der größtmöglichen Patientensicherheit, der in jedem Fall vor einer größeren unkontrolliert austretenden Insulinmenge geschützt sein muß. Die elektrischen Durchführungen 31 und 32 sind ebenfalls leckdicht ausgeführt, so daß auch hier weitgehende Sicherheit gegen austretende Flüssigkeit gegeben ist.

* * * * *

P a t e n t a n s p r ü c h e

1.   Vorrichtung zum Fördern und Dosieren kleinster Flüssigkeitsmengen, insbesondere zur Anwendung für den menschlichen oder tierischen Körper, bestehend aus einer piezokeramisch beschichteten Membran, die mit ihrem Rand allseitig an eine starre Wand angrenzt, wobei die so gebildete Pumpenkammer eine durch entsprechende Mittel verschließbare Durchlaßöffnung aufweist,

d a d u r c h   g e k e n n z e i c h n e t ,

daß die Durchlaßöffnung bei spannungsloser piezokeramischer Beschichtung (3, 4, 3', 4') durch die dabei an der starren Wand flach anliegende Membran (7, 7') unabhängig von den Verschlußmitteln der Durchlaßöffnung dichtend abgeschlossen ist,

daß die Membran eine Öffnung (11, 11') mit Sperrmitteln (9) aufweist, deren Durchlässigkeit entgegengesetzt zur Förderrichtung gering ist, bezogen auf die Durchlässigkeit der unverschlossenen Durchlaßöffnung (13, 13'), und

daß die Sperrmittel (9) entfernt von dem Ort der Durchlaßöffnung (13) bei flach anliegender Membran (7, 8) angeordnet sind.

2.   Vorrichtung nach Anspruch 1, d a d u r c h   g e k e n n z e i c h n e t ,   daß die Membran (7, 7') zweiseitig piezokeramisch beschichtet ist.

3.    Vorrichtung nach einem der Ansprüche 1 oder 2, d a - d u r c h   g e k e n n z e i c h n e t , daß jenseits der Durchlaßöffnung eine weitere, der ersten entsprechende piezokeramisch beschichtete Membran (8) derart angeordnet ist, daß die Durchlaßöffnung (13) bei spannungsloser piezokeramischer Beschichtung (5, 6) der weiteren Membran durch die dabei flach anliegende Membran dichtend abgeschlossen ist und daß elektrische Spannungserzeugermittel vorgesehen sind, welche an die Beschichtungen der Membranen (7, 8) zeitlich nacheinander abwechselnd auslenkende Spannungen abgeben.

4.    Vorrichtung nach Anspruch 1, d a d u r c h   g e - k e n n z e i c h n e t ,   daß die Verschlußmittel der Durchlaßöffnung aus einem selbstschließenden Ventil (15 bis 19) bestehen, bei dem ein beweglicher Dichtungskörper (17) einen Permanentmagneten enthält, auf den ein weiterer stationärer Permanentmagnet (18) eine Kraft in das Ventil schließender Richtung ausübt.

5.    Vorrichtung nach einem der vorangehenden Ansprüche, d a d u r c h   g e k e n n z e i c h n e t ,   daß die Sperrmittel (9) aus einem Strömungswiderstand für die zu fördernde Flüssigkeit bestehen.

6.    Vorrichtung nach Anspruch 5, d a d u r c h   g e - k e n n z e i c h n e t ,   daß der Strömungswiderstand durch ein Membranfilter gebildet wird.

7. Vorrichtung nach Anspruch 6, d a d u r c h   g e - k e n n z e i c h n e t ,   daß das Membranfilter aus PTFE mit einer Porosität von im wesentlichen 0,2/um besteht.

8.   Vorrichtung nach einem der vorangehenden Ansprüche, d a d u r c h   g e k e n n z e i c h n e t ,   daß die starre Wand den Abschluß eines Vorratsbehälters (1) bildet.

9.   Vorrichtung nach Anspruch 8, d a d u r c h   g e - k e n n z e i c h n e t ,   daß das Innere des Vorratsbehälters (1) im Betrieb unter Überdruck steht und und die Membran (7, 7') an seiner Innenseite angeordnet ist.

10.   Vorrichtung nach einem der Ansprüche 1 bis 7, d a - d u r c h   g e k e n n z e i c h n e t ,   daß die Öffnung der dem Vorratsbehälter (1) zugewandten Membran über einen flexiblen Schlauch mit dem Innern des unter Überdruck stehenden Vorratsbehälters verbunden ist.

11. Vorrichtung nach einem der Ansprüche 9 oder 10, d a - d u r c h   g e k e n n z e i c h n e t ,   daß der Vor- ratsbehälter flexibel ausgebildet ist und von einem unter Überdruck stehenden gasförmigen Medium umgeben ist.

*  *  *  *  *

FIG.1  FIG.2  FIG.3  FIG.4

# FIG.5

# FIG.6

# FIG.7

0025005

Europäisches Patentamt

EUROPÄISCHER RECHERCHENBERICHT

0025005
Nummer der Anmeldung

EP 80 73 0057

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| D | IEEE TRANS. ON SONICS AND ULTRA-SONICS, Band SU-25, Nr. 3, Mai 1975, Seiten 153-156 W.J. SPENCER et al.: "An Electronically Controlled Piezoelectric Insulin Pump and Valves" <br><br> * Seiten 153-154; Abbildung 4 * <br><br> -- | 1 |
| | US - A - 3 361 067 (WEBB) <br><br> * Abbildugen 1,2; Spalte 3, Zeilen 1-75; Spalte 4, Zeilen 1-52 * <br><br> -- | 1,2,5 |
| | US - A - 3 587 328 (SCHUEMANN) <br><br> * Abbildungen 1,4,5; Spalte 1, Zeilen 5-40; Spalte 2, Zeilen 69-75; Spalte 3, Zeilen 1-28 * <br><br> -- | 1 |
| P | MEDICAL & BIOLOGICAL ENGINEERING & COMP., Band 18, Nr. 4, Juli 1980, Seiten 527-537 Stevenage, G.B. W. SCHUBERT et al.: "An implantable artificial pancreas" <br><br> * Abbildungen 1-4; Seiten 527-530 * <br><br> -- | 1-11 |
| A | US - A - 3 107 630 (JOHNSON) | |
| A | US - A - 3 270 672 (HAINES) | |
| A | DE - A - 1 453 655 (PHYSICS INTERNATIONAL CO.) | |
| A | DE - A - 2 354 249 (AB ORIGINAL ODHNER) <br> ./. | |

**KLASSIFIKATION DER ANMELDUNG (Int Cl )**

H 01 L 41/08
F 04 B 43/04
A 61 M 5/14

**RECHERCHIERTE SACHGEBIETE (Int. Cl.)**

A 61 M
F 04 B
H 01 L
A 61 F

**KATEGORIE DER GENANNTEN DOKUMENTE**

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

Der vorliegende Recherchenbericht wurde für alle Patentansprüche erstellt.

| Recherchenort | Abschlußdatum der Recherche | Prüfer |
|---|---|---|
| Den Haag | 28-11-1980 | NOESEN |

EPA form 1503.1 06.78

**Europäisches Patentamt**

**EUROPÄISCHER RECHERCHENBERICHT**

| | EINSCHLÄGIGE DOKUMENTE | | KLASSIFIKATION DER ANMELDUNG (Int. Cl.³) |
|---|---|---|---|
| Kategorie | Kennzeichnung des Dokuments mit Angabe, soweit erforderlich, der Maßgeblichen Teile | betrifft Anspruch | |
| A | US - A - 3 763 385 (MOSSOTTI) | | |
| A | FR - A - 2 272 686 (ALZACORP.) | | |
| A | US - A - 3 606 592 (MADURSKI) | | |
| | ---- | | |
| | | | **RECHERCHIERTE SACHGEBIETE (Int. Cl.³)** |

EPA Form 1503.2 06.78